# FASCICULE DE BREVET EUROPEEN

(11) **EP 0 844 849 B1**
(45) Date de publication et mention de la délivrance du brevet: **10.01.2001**
(21) Numéro de dépôt: 95927601.5
(22) Date de dépôt: 03.08.1995
(51) Int. Cl.: A61B 17/68, A61F 2/30

(54) **CHEVILLE INTRAFOCALE**
INTRAFOKALER DÜBEL
INTRAFOCAL PIN

(43) Date de publication de la demande: 03.06.1998
(73) Titulaire: Persoons, Dominique, 62150 Rebreuve-Renchicourt (FR)
(72) Inventeur: Persoons, Dominique, 62150 Rebreuve-Renchicourt (FR)
(74) Mandataire: Kuborn, Jacques
(86) Numéro de dépôt international: BE9500072
(87) Numéro de publication internationale: WO9705830

(56) Documents cités:
- US-A- 4 969 888
- US-A- 5 006 120

## Description

L'invention concerne une cheville intrafocale pour la stabilisation et l'ostéosynthèse d'une fracture du poignet, en particulier avec déficit d'os cortical à la partie postéro-externe de la métaphyse du radius.

L'ostéosynthèse de la fracture du poignet dite "de Pouteau-Colles" reste difficile; il s'agit d'une véritable fracture du radius accompagnée d'un tassement de l'os. La réduction du déplacement ne restitue pas l'intégrité osseuse, et la fracture ne peut rester stable à cause du déficit d'os cortical sur la partie postéro-externe de la métaphyse du radius.

Plusieurs solutions ont déjà été proposées en vue de compenser ce "trou" situé sur la face postéro-externe du radius: brochage oblique selon Kapandji, brochage élastique selon Py et Demanet, fixateur externe en distraction, et greffe osseuse.

Ainsi, le brevet US n° 5 006 120 décrit un traitement d'une telle fracture par comblement du déficit osseux par une greffe osseuse ou de l'os artificiel, et maintien par une plaque et des vis métalliques.

Ces solutions présentent cependant des inconvénients: les broches sont affleurantes et peuvent provoquer des troubles cutanés ou tendineux; les broches élastiques sont parfois instables; les fixateurs externes engendrent des raideurs; et le traitement par greffe osseuse est exagérément lourd pour une fracture qui est très commune chez les personnes âgées.

L'objet de l'invention est donc de fournir un moyen permettant un traitement rapide et peu invasif, qui vise d'abord à assurer une réduction stable, en comblant la perte de substance osseuse sur le versant postérieur externe du radius.

Cet objet est atteint avec une cheville intrafocale dont le corps présente à une première extrémité une section plus grande que celle de sa seconde extrémité, et porte un organe de blocage définissant, au moins à ladite première extrémité, entre un premier et un deuxième éléments successifs, en saillie sensiblement radiale, une première encoche destinée à enclaver la paroi corticale postérieure de la métaphyse du radius; la section de ladite première extrémité du corps est destinée à compenser en substance le déficit osseux à la partie postéro-externe de la métaphyse du radius. Cette cheville est introduite dans le foyer de la fracture pour le redéployer, et elle traverse toute la largeur du radius pour en stabiliser au moins la paroi corticale postérieure, et de préférence également la paroi corticale antérieure.

Selon d'autres caractéristiques de l'invention:
- ledit organe de blocage présente de plus un troisième et un quatrième éléments successifs, en saillie sensiblement radiale, à ladite seconde extrémité dudit corps, définissant une seconde encoche destinée à enclaver la paroi corticale antérieure de la métaphyse du radius;
- lesdits éléments en saillie sont des éléments séparés entourant chacun au moins une fraction d'une section dudit corps;
- ledit corps présente à ladite première extrémité une tête de section supérieure à celle de ladite première extrémité, et constituant ledit premier élément en saillie;
- au moins une partie d'extrémité de certains élément en saillie dudit organe de blocage fait un angle aigu par rapport à l'axe longitudinal du corps;
- ledit organe de blocage entoure hélicoïdalement ledit corps;
- ledit organe de blocage s'étend sensiblement sur toute la longueur du corps, pour enclaver la paroi corticale à la partie postérieure et à la partie antérieure du radius, respectivement à la première et à la seconde partie d'extrémité du corps;
- l'espace libre entre deux spires successives de l'organe de blocage constitue lesdites encoches;
- les bords de l'organe de blocage sont des bords non tranchants;
- la base du corps est une base non perforante, mousse ou plate;
- la face externe de la tête est arrondie;
- le corps de la cheville est creux.
- le premier élément en saillie est mobile angulairement par rapport audit corps.

On connaît en chirurgie des vis prévues pour assurer une fixation dans un tissu osseux. Ces vis sont conçues pour pénétrer à force dans l'os, en s'y agrippant par leur filet hélicoïdal à bord tranchant.

Dans son mode de réalisation préféré, la cheville de l'invention se présente extérieurement sous une forme un peu semblable à une telle vis, avec un corps allongé et un filet hélicoïdal. Elle présente cependant des caractéristiques, et remplit une fonction essentiellement différentes, en particulier en ce qu'elle ne présente aucun effet de pénétration dans l'os, mais se glisse plutôt entre deux fragments osseux, et en ce qu'elle n'assure également par elle-même aucun effet de fixation des fragments osseux, comme le ferait une vis, mais agit plutôt comme un coin écartant des fragments osseux pressés l'un contre l'autre par les muscles et les tendons.

En plus de son effet de coin, assuré par un évasement longitudinal de son corps, la cheville assure également une stabilisation latérale de la fracture en fixant la paroi corticale de l'os fracturé, de part et d'autre de la fracture et de préférence tant à la face antérieure qu'à la face postérieure du radius, par un effet d'encoche de saillies périphériques.

D'autres aspects, caractéristiques et avantages de l'invention apparaîtront de la description qui suit, et du dessin annexé sur lequel:
La figure 1 est une vue de profil d'un mode de réalisation d'une cheville intrafocale selon l'invention,
La figure 2 est une vue de profil d'une variante du mode de réalisation de la figure 1,
La figure 3 est une vue profil d'une autre variante du mode de réalisation de la figure 1,
La figure 4 est une vue de profil d'une version simplifiée de la cheville de l'invention,
La figure 5 est une vue de profil d'une variante encore simplifiée du mode de réalisation de la figure 4,
Les figures 6 et 7 sont des vues d'artiste de variantes des figures 4 et 5, et
La figure 8 est une vue en coupe d'une variante à tête mobile.

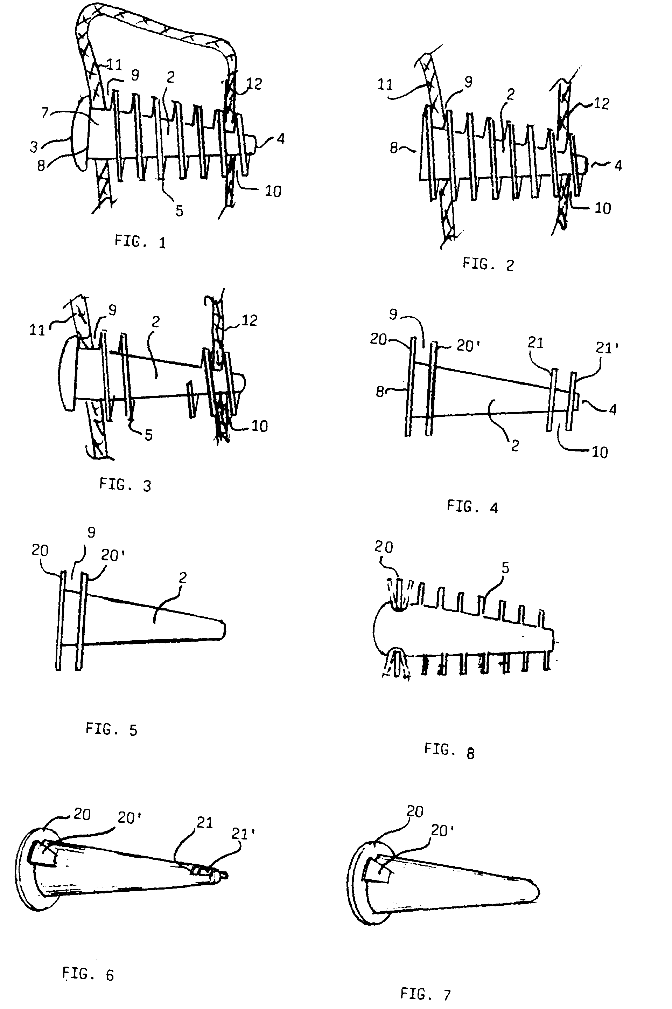

En se reportant à la figure 1, la cheville 1 est constituée d'un corps 2 portant à un extrémité 8 une tête 3, et pourvue à son extrémité opposée 4 d'une base non perforante, mousse ou plate. Le corps porte d'autre part un filet hélicoïdal 5 à bord non tranchant, plat ou mousse.

Le filet 5 se termine à distance de la tête 3, pour laisser libre une partie de col 7 du corps, contiguë à la tête. Comme on le comprendra, le bord terminal du filet 5 sera adouci (émoussé), aussi bien côté base 4 que côté tête 8, pour éviter de léser les tissus.

Au dessin, on a également schématisé en pointillés une portion de radius en coupe, pour illustrer l'utilisation de la cheville 1. Comme on le voit, la paroi corticale postérieure 11 vient s'enclaver dans une encoche 9 formée entre la tête 3 et la spire terminale du filet 5, tandis que la paroi corticale antérieure 12 vient s'enclaver dans une encoche 10 formée entre deux spires successives, côté base 4 de la cheville.

La tête 3 est séparée du col 7 par une marge abrupte, tandis que la génératrice du filet hélicoïdal est sensiblement perpendiculaire à l'axe longitudinal du corps.

Le diamètre du corps à l'endroit du col 7 est d'autre part supérieur à son diamètre côté base 4, pour assurer un effet de coin en vue d'ouvrir le foyer de fracture et de combler le déficit d'os cortical à la face postéro-externe du radius (côté tête de la cheville).

La face externe de la tête 3 est arrondie et lisse, pour éviter de léser les tissus mous environnant.

La base 4 est de même non pointue, pour les mêmes raisons; une base non pointue ne pose pas de problème particulier dans l'invention, puisque la cheville est prévue pour être insérée au foyer de la fracture, et qu'elle ne doit dès lors pas être perforante.

De même, le bord du filet hélicoïdal est non-tranchant, pour éviter des lésions aux tissus, tandis que le pas du filet hélicoïdal est tel que l'espace libre entre deux spires est suffisant pour y loger la paroi corticale du radius.

La section du col 7 comble le déficit osseux dû au tassement cortical postérieur, et permet la ré-expansion de la face postérieure du foyer de fracture.

Lors de la fracture, la paroi corticale antérieure ne subit d'autre part pas de tassement, de sorte que le diamètre du corps de la cheville à la base 4 peut être aussi faible que possible, compte tenu de ce qu'elle doit néanmoins présenter une section suffisante pour assurer un contact non traumatique avec les tissus environnants.

La section du corps 2 entre le col 7 et la base 4 peut être généralement quelconque, dans la mesure où elle ne joue aucun rôle lorsque la cheville est en position. De préférence cependant, le corps présentera un évasement longitudinal régulier, de façon à assurer une distraction progressive, et donc avec un effort régulier, du foyer de la fracture, au fur et à mesure de la progression de la cheville à travers le radius.

La cheville intrafocale selon l'invention peut éventuellement être creuse, en vue de son guidage par une broche-guide, sans que cela modifie le principe de son fonctionnement.

Cette cheville sera mise en place à partir de la face postérieure du radius, par rotation à la manière d'une vis, par exemple à l'aide d'un outil du type tournevis coopérant avec un logement prévu dans la tête 3, le filet 5 facilitant la progression de la cheville en s'appuyant sur la paroi corticale de l'os.

Dans la variante de la figure 2, la cheville ne comporte pas de tête, la paroi corticale postérieure 11 venant s'enclaver entre les deux spires terminales, côté extrémité 8, comme la paroi corticale 12 côté base 4.

Dans la variante de la figure 3, le filet est interrompu dans la partie centrale de la cheville. En effet, seules les parties terminales du filet servent à bloquer les parois corticales, de sorte que la partie centrale du filet ne remplit pas de fonction particulière, et peut être supprimée.

Dans la variante simplifiée et moins préférée de la figure 4, le corps sensiblement conique porte à chaque extrémité deux saillies périphériques contiguës 20, 20' et 21, 21', définissant les encoches destinées à enclaver la paroi corticale à chaque face du radius. Un inconvénient de ce mode de réalisation, est la distance fixe entre les deux encoches 9, 10 ainsi définies, qui ne permet pas d'adaptation en fonction de la taille du radius du patient traité.

Dans la variante encore simplifiée de la figure 5, seules deux saillies 20, 20', définissant une seule encoche 9 sont prévues. En effet, bien que ce soit utile pour répartir mieux les contraintes mécaniques, il n'est pas nécessaire d'enclaver la paroi corticale de part et d'autre du radius, tant qu'est atteint l'effet de compensation du déficit osseux à la face postérieure d'une part, et de blocage des deux morceaux de l'os fracturé vis-à-vis d'un glissement latéral d'autre part, et ceci est déjà atteint par la cheville de la figure 5.

Encore un inconvénient des modes de réalisation des figures 4 et 5 est qu'ils nécessitent d'écarter les lèvres de la fracture, préalablement à la mise en place de la cheville, pour permettre le passage des éléments en saillie, et que l'implant ne peut être retiré ultérieurement.

Enfin, les variantes des figures 6 et 7 ont pour but de montrer que les saillies définissant les encoches d'enclavement des parois corticales ne doivent pas entourer complètement la section du corps, mais peuvent au contraire avoir une extension périphérique limitée, facilitant également la mise en place de la cheville, puisque celle-ci peut être insérée avec les saillies 20', 21 et 21' sensiblement parallèles à la fracture, la cheville étant ensuite pivotée de 90° pour assurer le blocage, à la manière d'une fixation à baïonnette.

Les éléments en saillie discontinus 20', 21, 21', ou au moins leur partie d'extrémité, peut être inclinée suivant un angle aigu par rapport à l'axe longitudinal de la cheville (à la manière du filet hélicoïdal 5), pour faciliter encore leur passage au-delà de la paroi corticale.

Dans la variante des figures 6 et 7, la saillie 20 a été maintenue continue pour assurer un appui maximum pour la paroi corticale endommagée à la partie postérieure du radius, Elle pourrait cependant au besoin également ne s'étendre que sur une partie de la périphérie, comme la saillie 20'.

Enfin, dans le mode de réalisation de la figure 8, l'élément formant saillie 20 est mobile pour lui permettre de s'adapter dans une certaine mesure à l'inclinaison de la paroi corticale (les deux positions extrêmes étant représentées en pointillés).

A titre de variante complémentaire, il est bien entendu évident que la portion de filet du côté de la base 4 pourrait également être supprimée dans le mode de réalisation de la figure 3, pour aboutir à un mode de réalisation analogue à celui des figures 5 et 7.

Enfin, dans les modes de réalisation des figures 4 à 7, on pourrait encore avantageusement disposer les saillies 20', 21 et 21', non pas perpendiculairement par rapport à l'axe du corps 2 de la cheville, mais bien en oblique par rapport à celui-ci, de façon à faciliter l'insertion.

En ce qui concerne les dimensions de la cheville, celles-ci doivent bien sûr être adaptées à la taille du radius fracturé. A titre d'exemple, on peut cependant mentionner les dimensions suivantes:
- marge tête-col: 9,5 ± 1,5 mm (diamètre ou largeur)
- diamètre du col: 6,5 ± 1,5 mm
- longueur du corps: 26 ± 6 mm
- diamètre de la base: 2,5 ± 1,5 mm
- hauteur du filet: 2,2 ± 1,2 mm

Bien entendu, l'invention n'est pas limitée aux modes de réalisation représentés et décrits, qui n'ont été choisis qu'à titre d'exemple.

## Revendications

1. Cheville intrafocale pour fracture du poignet, prévue pour traverser toute la largeur du radius et comprenant un corps (2) avec une première (8) et une seconde (4) extrémités, cheville dans laquelle le corps (2) de la cheville présente à la première extrémité (8) une section plus grande que celle de la seconde extrémité (4), et le corps (2) présente un organe de blocage (3, 5; 5; 20, 20') définissant, au moins à ladite première extrémité (8), entre un premier et un deuxième éléments successifs, en saillie sensiblement radiale, une première encoche (9) destinée à enclaver la paroi corticale (11) postérieure de la métaphyse du radius, tandis que la section de ladite première extrémité (8) du corps est destinée à compenser en substance le déficit osseux à la partie postéro-externe de la métaphyse du radius.

2. Cheville intrafocale selon la revendication 1, caractérisée en ce que ledit organe de blocage présente de plus un troisième et un quatrième éléments successifs, en saillie sensiblement radiale, à ladite seconde extrémité (4) dudit corps, définissant une seconde encoche (10) destinée à enclaver la paroi corticale antérieure (12) de la métaphyse du radius.

3. Cheville intrafocale selon la revendication 1 ou 2, caractérisée en ce que lesdits éléments en saillie sont des éléments séparés (20, 20': 21, 21') entourant chacun au moins une fraction d'une section dudit corps.

4. Cheville intrafocale selon l'une quelconque des revendications 1 à 3, caractérisée en ce que ledit corps présente à ladite première extrémité (8) une tête (3) de section supérieure à celle de ladite première extrémité, et constituant ledit premier élément en saillie.

5. Cheville intrafocale selon l'une quelconque des revendications précédentes, caractérisée en ce qu'au moins une partie d'extrémité de certains élément en saillie dudit organe de blocage fait un angle aigu par rapport à l'axe longitudinal du corps.

6. Cheville intrafocale selon la revendication 1, caractérisé en ce que ledit organe de blocage (5) entoure hélicoïdalement ledit corps (2).

7. Cheville intrafocale selon la revendication 6, caractérisée en ce que ledit organe de blocage (5) s'étend sensiblement sur toute la longueur du corps, pour enclaver la paroi corticale à la partie postérieure (11) et à la partie antérieure (12) du radius, respectivement à la première (8) et à la seconde (4) partie d'extrémité du corps.

8. Cheville intrafocale selon l'une quelconque des revendications 6 et 7, caractérisée en ce que l'espace libre entre deux spires successives de l'organe de blocage (5) constitue lesdites encoches (9, 10).

9. Cheville intrafocale selon l'une quelconque des revendications précédentes, caractérisée en ce que les bords de l'organe de blocage (5) sont des bords non tranchants.

10. Cheville intrafocale selon l'une quelconque des revendications précédentes, caractérisée en ce que la base (4) du corps (2) est une base non perforante, mousse ou plate.

11. Cheville intrafocale selon l'une quelconque des revendications précédentes, caractérisée en ce que la face externe de la tête (3) est arrondie.

12. Cheville intrafocale selon l'une quelconque des revendications précédentes, caractérisée en ce que le corps (2) de la cheville est creux.

13. Cheville intrafocale selon l'une quelconque des revendications précédentes, caractérisée en ce que le premier élément en saillie (20) est mobile angulairement par rapport audit corps (2).

## Patentansprüche

1. Intrafokaler Dübel für Handgelenkfrakturen, der zum Hindurchgehen durch die gesamte Radiusbreite vorgesehen ist und einen Körper (2) mit einem ersten (8) und einem zweiten (4) Ende umfaßt, wobei bei dem Dübel der Körper (2) des Dübels am ersten Ende (8) einen Querschnitt aufweist, der größer ist als derjenige des zweiten Endes (4), und der Körper (2) ein Sperrorgan (3, 5; 5; 20, 20') aufweist, das zumindest an dem ersten Ende (8) zwischen einem ersten und einem zweiten aufeinanderfolgenden Element radial merklich auskragend eine erste Nut (9) definiert, die dazu bestimmt ist, die hintere Schalenwand (11) der Metaphyse des Radius einzuschließen, während der Querschnitt des ersten Endes (8) des Körpers dazu bestimmt ist, das Knochendefizit am posteroexternen Teil der Metaphyse des Radius zu kompensieren.

2. Intrafokaler Dübel nach Anspruch 1, dadurch gekennzeichnet, daß das Sperrorgan am zweiten Ende (4) des Körpers ferner dritte und vierte radial merklich auskragende, aufeinanderfolgende Elemente aufweist und eine zweite Nut (10) definiert, die dazu bestimmt ist, die vordere Schalenwand (12) der Metaphyse des Radius einzuschließen.

3. Intrafokaler Dübel nach Anspruch 1 oder 2, dadurch gekennzeichnet, daß die auskragenden Elemente gesonderte Elemente (20, 20'; 21, 21') sind, die jeweils zumindest einen Teil eines Querschnitts des Körpers umgeben.

4. Intrafokaler Dübel nach einem der Ansprüche 1 bis 3, dadurch gekennzeichnet, daß der Körper an dem ersten Ende (8) einen Kopf (3) mit einem Querschnitt aufweist, der größer ist als der des ersten Endes, und das erste auskragende Element bildet.

5. Intrafokaler Dübel nach einem der vorhergehenden Ansprüche, dadurch gekennzeichnet, daß zumindest ein Endteil einiger auskragender Elemente des Sperrorgans einen spitzen Winkel bezüglich der Längsachse des Körpers bildet.

6. Intrafokaler Dübel nach Anspruch 1, dadurch gekennzeichnet, daß das Sperrorgan (5) den Körper (2) spiralförmig umgibt.

7. Intrafokaler Dübel nach Anspruch 6, dadurch gekennzeichnet, daß das Sperrorgan (5) sich ziemlich genau über die gesamte Länge des Körpers erstreckt, um die Schalenwand am hinteren Teil (11) und am vorderen Teil (12) des Radius bzw. am ersten (8) und zweiten (4) Endteil des Körpers einzuschließen.

8. Intrafokaler Dübel nach einem der Ansprüche 6 und 7, dadurch gekennzeichnet, daß der freie Raum zwischen zwei aufeinanderfolgenden Windungen des Sperrorgans (5) die Nuten (9, 10) bildet.

9. Intrafokaler Dübel nach einem der vorhergehenden Ansprüche, dadurch gekennzeichnet, daß die Ränder des Sperrorgans (5) nicht schneidende Ränder sind.

10. Intrafokaler Dübel nach einem der vorhergehenden Ansprüche, dadurch gekennzeichnet, daß die Basis (4) des Körpers (2) eine nicht perforierende, stumpfe oder flache Basis ist.

11. Intrafokaler Dübel nach einem der vorhergehenden Ansprüche, dadurch gekennzeichnet, daß die Außenseite des Kopfes (3) abgerundet ist.

12. Intrafokaler Dübel nach einem der vorhergehenden Ansprüche, dadurch gekennzeichnet, daß der Körper (2) des Dübels hohl ist.

13. Intrafokaler Dübel nach einem der vorhergehenden Ansprüche, dadurch gekennzeichnet, daß das erste auskragende Element (20) bezüglich des Körpers (2) winkelbeweglich ist.

## Claims

1. Intrafocal peg for wrist fracture, adapted to pass through the full width of the radius and comprising a body (2) with a first (8) and a second (4) ends, peg in which the peg body (2) includes at the first end (8) a cross-sectional area larger than that of the second end (4), and the body (2) has a blocking member(3, 5 ; 5 ; 20, 20') defining, at least at said first end (8), between first and second sequential, substantially radially protruding members, a first notch (9) intended for housing the posterior cortical wall (11) of the metaphysis of the radius, while the cross sectional area of said first end (8) of the body is intended for substantially compensating the bone defect at the postero-external part of the metaphysis of the radius.

2. Intrafocal peg according to claim 1, characterized in that said blocking member additionnally includes third and fourth sequential, substantially radially protruding members at said second end (4) of said body, defining a second notch (10) intended for housing the anterior cortical wall (12) of the metaphysis of the radius.

3. Intrafocal peg according to claims 1 or 2, characterized in that said protuding members are individual members (20, 20'; 21, 21'), each surrounding at least part of a section of said body.

4. Intrafocal peg according to anyone of claims 1 to 3, characterized in that said body includes, at said first end (8), a head (3) of a cross sectional area larger than that of said first end, and constituting said first protuding member.

5. Intrafocal peg according to anyone of the preceding claims, characterized in that at least a terminal part of some protuding members of said blocking member makes an acute angle with the longitudinal axis of the body.

6. Intrafocal peg according to claim 1, characterized in that said blocking member (5) helically surrounds said body (2)

7. Intrafocal peg according to claim 6, characterized in that said blocking member (5) extends substantially over the full length of the body, for housing the cortical wall at the posterior part (11) and at the anterior part (12) of the radius, at the first (8) and at the second (4) terminal parts of the body, respectively.

8. Intrafocal peg according to anyone of claims 6 and 7, characterized in that the free space between two successive whorls of the blocking member (5) constitutes said notches(9, 10)

9. Intrafocal peg according to anyone of the preceding claims characterized in that the edges of the blocking member (5) are non cutting edges.

10. Intrafocal peg according to anyone of the preceding claims characterized in that the base (4) of the body (2) is a non perforating, blunt or flat base.

11. Intrafocal peg according to anyone of the preceding claims, characterized in that the external face of the head (3) is rounded.

12. Intrafocal peg according to anyone of the preceding claims, characterized in that the body (2) of the peg is hollow.

13. Intrafocal peg according to anyone of the preceding claims, characterized in that the first protuding member (20) is angularly movable relative to said body (2).
